# EUROPEAN PATENT SPECIFICATION

(11) **EP 3 054 874 B1**
(45) Date of publication and mention of the grant of the patent: **20.11.2019**
(21) Application number: 14824956.8
(22) Date of filing: 09.10.2014
(51) Int. Cl.: A61B 17/17, A61B 17/34, A61B 17/16, A61B 17/88

(54) **THREAD FORMING JAMSHIDI ASSEMBLY**
GEWINDEFORMENDE JAMSHIDI-ANORDNUNG
ENSEMBLE JAMSHIDI FORMANT UN FILET

(30) Priority: 09.10.2013 US 201361888847 P
(43) Date of publication of application: 17.08.2016
(73) Proprietor: Orthovita, Inc., Malvern, PA 19355 (US)
(72) Inventor: GEIST, Wyatt, Drake, Davie, FL 33328 (US)
(74) Representative: Regimbeau
(86) International application number: PCT/US2014/059933
(87) International publication number: WO 2015/054514

(56) References cited:
- US-A1- 2007 255 282
- US-A1- 2009 138 043
- US-A1- 2010 049 206
- US-A1- 2011 054 537
- US-A1- 2011 152 866
- US-A1- 2011 238 069
- US-A1- 2012 226 301

## Description

### FIELD OF THE INVENTION

The present invention relates to surgical instruments used for spinal surgery; more particularly to a thread forming Jamshidi assembly used in spinal surgery.

### BACKGROUND OF THE INVENTION

The central nervous system, made primarily of the brain and the spine, is a vital part of the human physiology responsible for coordinating many aspects of human activity. The spinal cord is made up of a bundle of nerve tissue and acts as a conduit to communicate neuronal signals from the brain to the rest of the body. Protecting the spinal cord is the spinal, or vertebral, column. Anatomically, the spinal column is made up of several regions, including the cervical, thoracic, lumbar and sacral regions, each containing a plurality of vertebrae. Medical procedures involving the vertebrae are normally complicated because of the preciseness and accuracy required to avoid both neural damage and injury to major blood vessels.

While most people have fully functional spinal cords, it is not uncommon for individuals to suffer some type of spinal ailment. For example, spinal fractures, or vertebra compression fractures, occur when one of the bones of the spinal column fractures. Depending on the injury, various procedures have been developed to provide relief.

Many spinal surgical produces, such as minimally invasive percutaneous techniques, use various hardware devices, such as metallic screws, such as pedicle screws, rods, plates, or cages. U.S. Patent 7,780,706 is an illustrative example of a pedicle screw assembly having a cannulated pedicle screw. As part of the surgical procedures, the pedicle screw gets passed over a previously placed guide wire. While such technique is relatively safe and effective, several possible problems are known to exist. For example, using a guide wire, while relatively safe, exposes the patient to risk of surgical complications. First, guide wires may advance through softer cancellous bone, resulting in severe damage to organs or vessels. Second, guide wires tend to travel a great distance during placement. Such increased distance can result in formation of kinks. Use of guide wires cause increased length of surgical instruments. The increased length makes the instruments more cumbersome, particularly when moving around fluoroscopic imaging devices, such as C-arm, which are critical for percutaneous screw instrumentation.

Accordingly, what is needed in the art is a device that can aid a surgeon in safely delivering pedicle screws to the patient.

US 2012/226301 A1 discloses a jamshidi assembly comprising an inner cannulated member with a threaded portion, a stylet and an outer sleeve member including an external threading.

### SUMMARY OF THE INVENTION

The present invention is directed towards a Jamshidi assembly as claimed in claim 1.

Accordingly, it is an objective of the present invention to teach a Jamshidi assembly which can be used to aid a user during a surgical procedure.

It is a further objective of the present invention to teach a Jamshidi assembly which can be used to aid a user during insertion of pedicle screws.

It is yet another objective of the present invention to teach a Jamshidi assembly which contains an outer sheath member adapted to rotate about an inner cannula member.

It is a still further objective of the present which contains an outer sheath member adapted to move about an inner cannula and/or a longitudinal axis in a linear manner

Other objectives and advantages of this invention will become apparent from the following description taken in conjunction with any accompanying drawings wherein are set forth, by way of illustration and example, certain embodiments of this invention. Any drawings contained herein constitute a part of this specification and include exemplary embodiments of the present invention and illustrate various objects and features thereof.

### BRIEF DESCRIPTION OF THE FIGURES

Figure 1 is an illustrative example of a Jamshidi assembly in accordance with the present invention;
Figure 2 is a side view of the Jamshidi assembly illustrated in Figure 1;
Figure 3 is a cross sectional view of the thread forming Jamshidi assembly taken along lines 2A-2A in Figure 2;
Figure 4 is an exploded view of the forming Jamshidi assembly;
Figure 5A is a partial view of an illustrative working end of the Jamshidi assembly, illustrating an embodiment of the working end as a reamer;
Figure 5B is an alternative embodiment view of a working end of the Jamshidi assembly;
Figure 6 is an illustrative embodiment of the medical instrument having a sharply pointed end;
Figure 7 is an illustrative embodiment of the outer sleeve member of the Jamshidi assembly;
Figure 8 is a partial view of the outer sleeve member, illustrating an alternative embodiment of the working end having a tapered distal end;
Figure 9 is a partial view of the distal end of the outer sleeve member, illustrating an alternative embodiment of the working end having a multiple crown end;
Figure 10 is a partial view of the distal end of the outer sleeve member illustrating an alternative embodiment of the working end having a tap;
Figure 11 is a partial view of an alternative embodiment of the Jamshidi assembly;
Figure 12 is a schematic representation of the Jamshidi assembly inserted within a patient;
Figure 13 is a schematic representation of the Jamshidi assembly in which the outer cannula is rotated, advancing in a linear manner about the inner cannula, thereby driving a working end into a vertebral body;
Figure 14 is a schematic representation of the Jamshidi assembly in which the working member is removed from the vertebral body, thereby exposing a threading formed therein.

### DETAILED DESCRIPTION OF THE INVENTION

While the present invention is susceptible of embodiment in various forms, there is shown in the drawings and will hereinafter be described a presently preferred, albeit not limiting, embodiment with the understanding that the present disclosure is to be considered an exemplification of the present invention and is not intended to limit the invention to the specific embodiments illustrated.

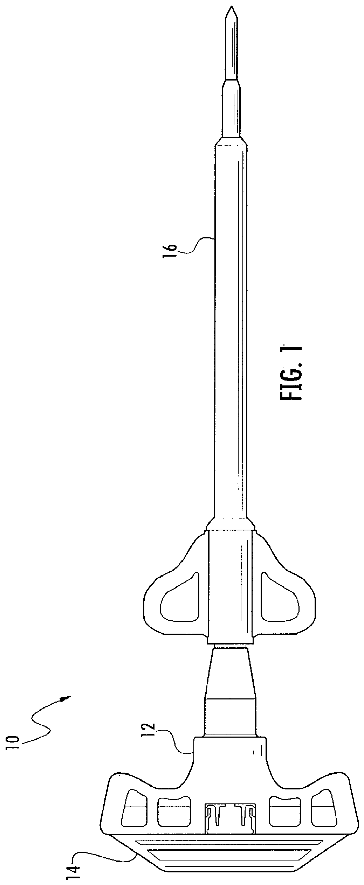

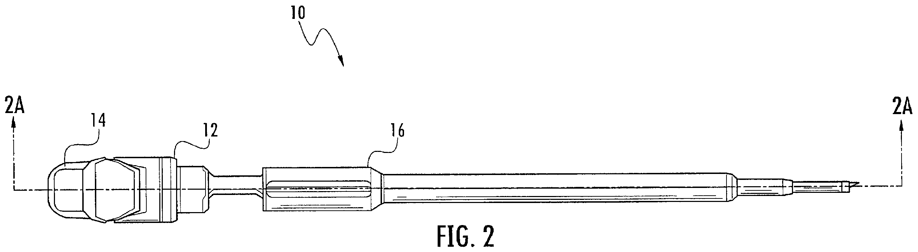

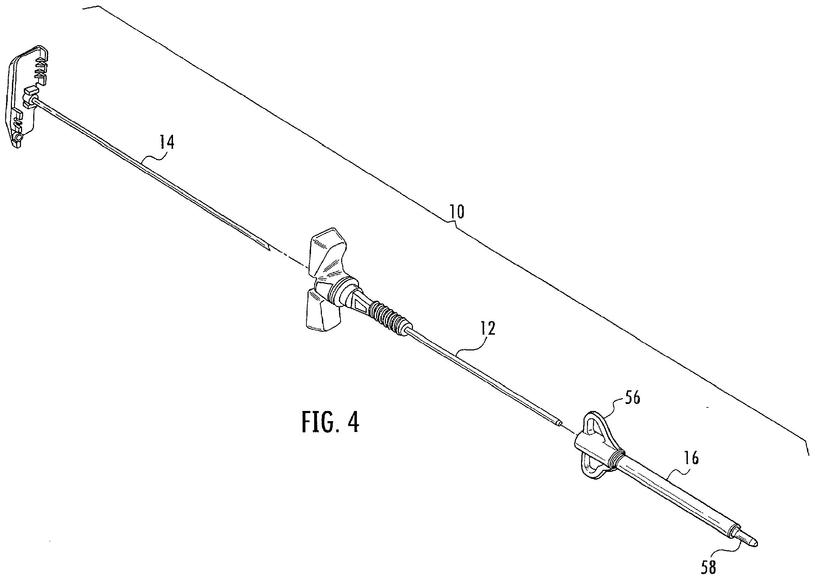

Referring to Figures 1-8, a Jamshidi type device having an outer sleeve with a working end, referred to generally as a Jamshidi assembly 10, is illustrated. The Jamshidi assembly 10 contains an inner cannulated member 12 sized and shaped to receive a sharp pointed medical device 14, and an outer sleeve member 16. The inner cannulated member 12 is slidably disposed within the outer sleeve member 16 thereby forming a coaxial relationship. When engaged with the inner cannulated member 12, the outer sleeve member 16 is constructed to rotate about and/or move linearly about a longitudinal axis of the Jamshidi assembly 10.

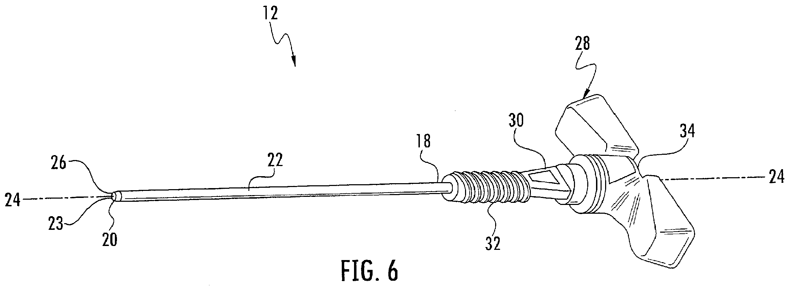

Referring to Figure 6, the cannulated member 12 comprises a first end 18, a second end 20, and a main body 22. The main body 22 is preferably an elongated body having a generally cylindrical shape. Additionally, the main body 22 is cannulated having a hollow interior 23 sized and shaped to receive and allow passage of other devices, such as, for example, the sharp pointed medical device 14, or a guide wire (not shown), along its longitudinal axis 24. The second end 20 contains opening 26 which allows the other devices described above to extend out past the main body 22. Attached to the first end 18 is an inner cannulated member handle 28 adapted for gripping with a hand. The inner cannulated member handle 28 contains a collar 30 fixedly secured to the main body 22 as well as a threaded sleeve 32. The threaded sleeve 32 is designed to engage with a portion of the outer sleeve member 16. Preferably, the inner cannulated member handle 28 contains an ergonomic shape to provide a comfort fit for the user. The inner cannulated member handle 28 also contains opening 34 to allow for insertion and passage of various medical devices into and within the interior of the main body 22.

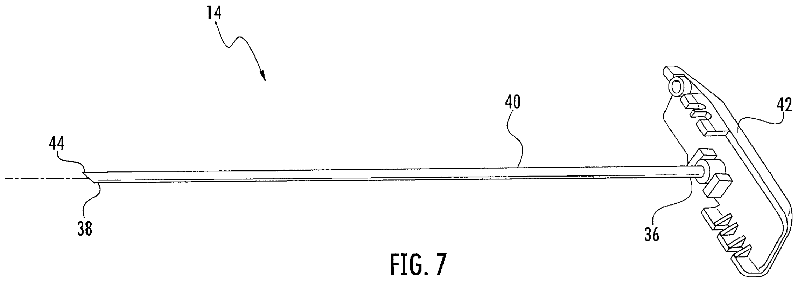

Referring to Figure 7, an illustrative example of the sharp pointed medical device 14, herein as a stylet, is shown. The stylet 14 comprises a first end 38, a second opposing end 38, and a stylet elongated body 40 there between. The first end 36 contains a handle 42 which is constructed and arranged to be gripped by a user's hand. The handle 42 is also designed to engage and secure to the inner cannula member handle 28 so that when the stylet 14 is inserted into the interior, hollow portion 23 of the inner cannula member 12, it can be locked in place if required. The second opposing end 38 contains a pointed end, illustrated herein as a trocar-tapered stylet tip 44, adapted to puncture a body component, such as a vertebral body or other bone structure. The pointed end 44 may include for example, a standard pointed tip having two or more edges, or a single or multi-beveled tip, such as a bi-beveled or tri-beveled tip. Additionally, the second opposing end 38 may be constructed to contain threading.

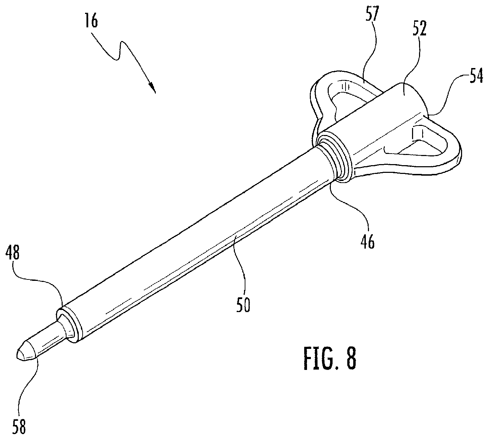

Referring to Figure 8, an illustrative embodiment of the outer sleeve member 16 is illustrated. The outer sleeve member 16 contains a first end 46, a second end 48, and an outer sleeve member main body 50. The outer sleeve member main body 50 is preferably cannulated, having an interior hollow portion sized and shaped to slideably engage with the inner cannula member main body 22. Coupled to, or integrally formed with the first end 46 is an inner cannula member receiving portion, illustrated herein as a cylindrical body 52.

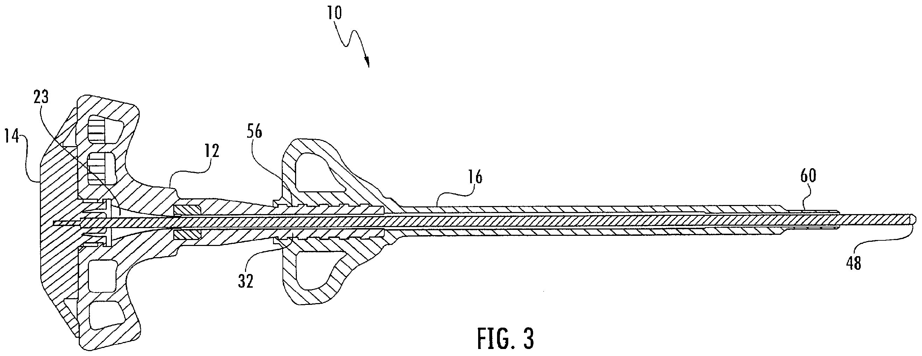

The cylindrical body 52 is sized and shaped to receive and engage with at least a portion of the inner cannulated member 12. Accordingly, the cylindrical body 52 contains opening 54 which exposes an interior portion that contains threading 56 (see Figure 3). The threading 56 (preferably an internal, female threading) of the cylindrical body 52 provides a mechanism to engage with the threading (external, male threading) of the threaded sleeve 32. As such, when the outer sleeve member 16 slidably engages with the inner cannulated member 12 to form a coaxial relationship in which the threaded sleeve 32 engages with the threading 56, the outer sleeve member 14 can be linearly moved along a longitudinal axis in a controlled manner. To aid the user in accomplishing such movement, the cylindrical body 52 contains a handle 57 sized and shaped to allow a user to grip it and to provide user leverage when rotating the outer sleeve member 16. While the preferred embodiment of the outer sleeve member 16 contains threading 56, alternative not claimed embodiments of the Jamshidi assembly 10 may include an outer sleeve member 16 that does not contain the treading.

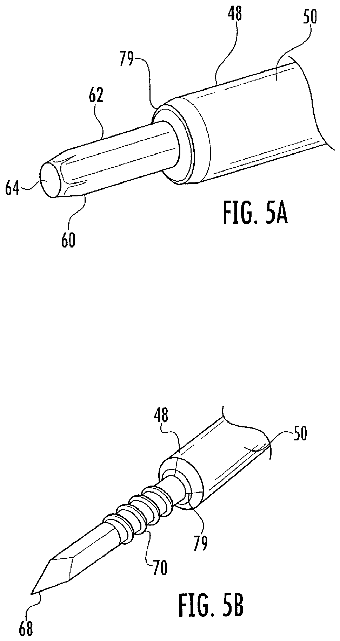

Positioned on the second end 48 is a working member 58. The working member 58 may be constructed as a separate, independent unit subsequently secured to the second end 48. Alternatively, the working member 58 can be integrally formed with and as part of the outer sleeve member main body 50. Figure 5A shows an embodiment of the working member illustrated as a reamer 60 having strait flutes 62. Alternatively, the reamer 60 may contain spiral flutes or helical flutes. The reamer 60 contains opening 64 to allow the outer sleeve member 14 to move about the inner cannulated member 12. The leading edge and outer diameter of the flutes 62 are constructed to size the hole formed in the bone for tapping or for thread forming screws without he need to place a separate tool into the formed hole. Extended surface 79 provides a stop to prevent the tool from being over inserted into the bone. Figure 5B illustrates an alternative embodiment of the working member 58. The working member 58 is shown as a tapping device comprising a pointed point end, such as a conical tip 68 and a threaded portion 70. In use, the conical tip 68 can be used to insert into a portion of the vertebral body and the threaded portion 70 can be used formation of conformations such as threading within the vertebral body, thereby aiding the insertion of a pedicle screw. Preferably, the thread pitch (distance from the crest of one thread to the next) and/or the lead (distance along the screw's axis that is covered by one complete rotation of the screw, 360 degrees) of the inner cannula threaded sleeve 32 is the same thread pitch and/or lead as threaded portion 70. In this manner, as the outer sleeve 16 is rotated, it moves along the inner cannula members 12 at the same rate or distance as the threaded portion 70 moves within the vertebral body. Alternatively, the threaded sleeve 32 may be designed to have different thread pitch and/or lead as that of the threaded portion 70. In addition, the threading 56 of the outer sleeve member may be constructed with the same thread pitch and/or lead as that of the working end 60 and/or other devices that contain threading, such as a tapping device in order to work in unison when in use. Alternatively, the threading 56 of the outer sleeve member 16 may have a different thread pitch and/or lead than the thread pitch of the working end 60 and/or other devices that contain threading. The threaded portions of the threaded sleeve and the working member according to the invention are defined in claim 1.

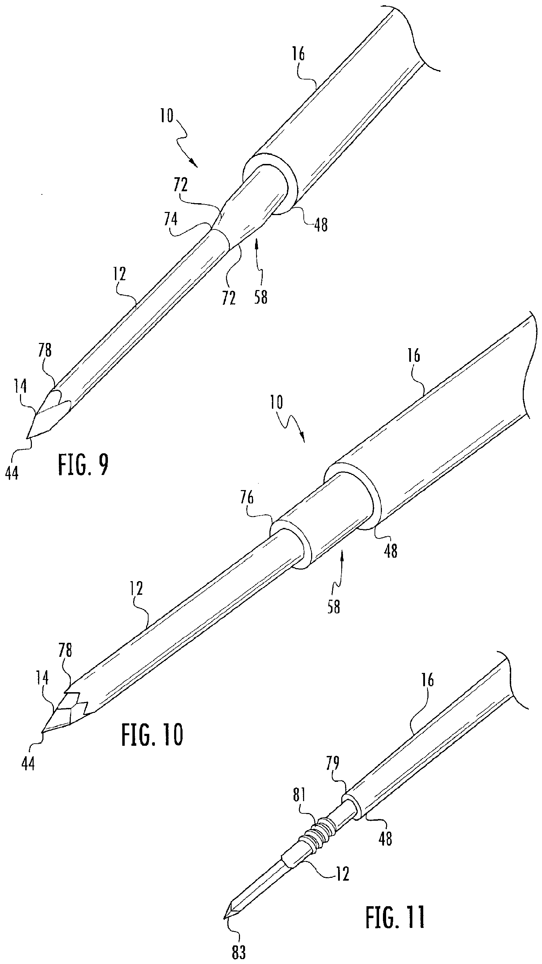

Figures 9 and 10 illustrate alterative embodiments of the Jamshidi assembly 10. The Jamshidi assembly 10 contains the same features as described above, differing mainly in the construction of the working member 58 of the outer sleeve member 16. As illustrated in Figure 9, the working member 58 of the outer sleeve member 16 is shown having a tapered end 72 terminating in a generally smooth distal end surface 74. Figure 10 illustrates the working member 58 of the outer sleeve member 16 having a triple crown cannula tip 76. The working member 58 may contain alternative surfaces having penetrating and/or advancement features, such as, but not limited to serrations or saw tooth ends. The triple crown cannula tip 76, along with the trocar-tapered stylet tip 44, and/or the multiple crown cannulated tip 78 of the inner cannula 12 provides a sharp, effective cutting edge designed for cortical penetration and medullary advancement which does not require much force from the surgeon.

Figure 11 illustrate an alternative embodiment of the Jamshidi assembly 10. In the embodiment illustrated, the distal end 48 of the outer sleeve member 16 contains a tapered lip or extended surface 79 which can be used as a stop measure during insertion and/or removal. In addition, the Jamshidi assembly 10 is shown having inner cannula member 12 having threaded portion 81 and a pointed end 83. The threading can be designed to have the same thread pitch and/or lead as any thread pith and/or lead associated with other components of the Jamshidi assembly 10.

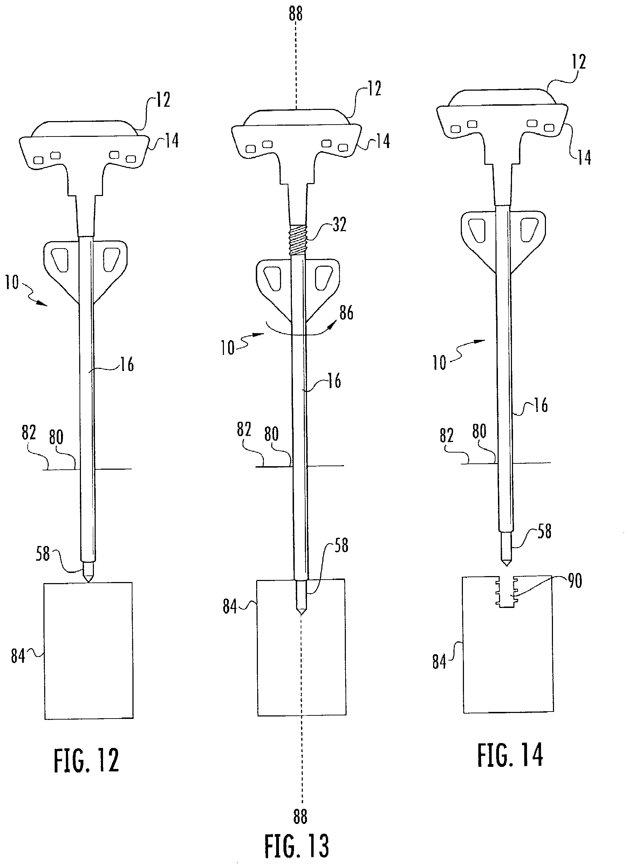

In use, the patient's skin and facia are incised according to standard medical protocol. The muscular tissues may be dissected as required. The Jamshidi assembly 10 is inserted into the incision and docked at the proper place. As illustrated in Figure 12, outer sleeve member 16, the inner cannula 12, and/or the stylet 16 is passed through an incision 80 through a patient's skin 82, and rests along a portion of vertebral body 84 (for example, starting point for a pedicle screw). As the user rotates the outer sleeve member 16, see arrow 86, the outer sleeve member 16 rotates about the threaded sleeve 32 of the inner cannula 12. Such rotation allows the outer sleeve member 16 to traverse about the inner cannula 12 along the longitudinal axis 88 of the Jamshidi assembly. As the outer sleeve member 16 moves in a linear manner towards the vertebral body 84, the working end 58 eventually contacts the vertebral body 84. The outer sleeve member 16 is further rotated, driving the working end 58 into the vertebral body 84, see Figure 13. The Jamshidi assembly 10 can be removed once the outer sleeve member 16 is rotated counterclockwise, back to its original starting point. Depending on the working end 58, some type of configuration, such as threading to aid in pedicle screw insertion, illustrated as partial dotted box 90 (see Figure 14) is cut into the vertebral body 84.

It is to be understood that while a certain form of the invention is illustrated, it is not to be limited to the specific form or arrangement herein described and shown. It will be apparent to those skilled in the art that various changes may be made without departing from the scope of the invention and the invention is not to be considered limited to what is shown and described in the specification and any drawings/figures included herein.

One skilled in the art will readily appreciate that the present invention is well adapted to carry out the objectives and obtain the ends and advantages mentioned, as well as those inherent therein. The embodiments, methods, procedures and techniques described herein are presently representative of the preferred embodiments, are intended to be exemplary and are not intended as limitations on the scope. Changes therein and other uses will occur to those skilled in the art which are encompassed within the invention and are defined by the scope of the appended claims. Although the invention has been described in connection with specific preferred embodiments, it should be understood that the invention as claimed should not be unduly limited to such specific embodiments. Indeed, various modifications of the described modes for carrying out the invention which are obvious to those skilled in the art are intended to be within the scope of the following claims.

## Claims

1. A jamshidi assembly (10) comprising:
an inner cannulated member (12) including a first end (18), a second end (20), and a main body portion (22), an inner cannulated member handle (28) adapted for gripping with a hand being secured to said first end (18) thereof, said inner cannulated handle member (28) including an opening (34) positioned to allow insertion of various medical devices through an interior passage of said inner cannulated member, a threaded sleeve (32) secured about said main body portion (22) of said inner cannulated member (12),
a stylet (14) including a first end (36), a second end (38) and an elongated body (40) there between, said elongated body (40) sized to fit through said interior passage sufficiently for said second end (38) of said stylet (14) to protrude from said second end (20) of said inner cannulated member (12) when inserted through said opening (34) thereof,
an outer sleeve member (16), said outer sleeve member (16) including a first end (46), a second end (48) and a main body portion (50), said outer sleeve member (16) including internal threading (56), said outer sleeve member (16) being cannulated to include an interior hollow portion, said interior hollow portion threadably engaged to an outer diameter of said main body portion (22) of said inner cannulated member (12) by engaging said internal threading (56) with the threading of said threaded sleeve (32), said second end (48) of said outer sleeve member (16) including a working member (58), wherein said working member (58) is constructed and arranged to increase the size of an aperture created by said interior hollow portion and is a tapping device having a pointed point end and a threaded portion (70), said threaded sleeve (32) having a thread pitch that is the same as a thread pitch of said threaded portion (70), said first end (46) of said outer sleeve member (16) including an outer sleeve handle (57),
whereby rotation of said outer sleeve handle (57) causes linear movement of said outer sleeve member (16) with reference to said inner cannulated member (12) such that said outer sleeve member (16) penetration into a bone member (84) is adjustably controlled by rotation of outer sleeve handle (57) with the linear movement of said outer sleeve member (16) with reference to said inner cannulated member (12) being at the same rate or distance as said threaded portion (70) moves within the bone member (84).

2. The jamshidi assembly (10) of claim 1, wherein said tapping device comprises a conical tip (68).

3. The jamshidi assembly (10) of claim 1, wherein said outer sleeve member (16) can be linearly moved along a longitudinal axis (24) of said inner cannulated member (12) in a controlled manner by rotating said outer sleeve member (16).

## Patentansprüche

1. Jamshidi-Anordnung (10), umfassend:
ein inneres durchbohrtes Element (12), das ein erstes Ende (18), ein zweites Ende (20) und einen Hauptkörperabschnitt (22), einen inneren durchbohrten Elementgriff (28) umfasst, der zum Greifen mit einer Hand geeignet ist, die am ersten Ende (18) gesichert ist, wobei das innere durchbohrte Griffelement (28) eine Öffnung (34) aufweist, die so positioniert ist, dass sie das Einsetzen verschiedener medizinischer Vorrichtungen durch einen inneren Durchgang des inneren durchbohrten Elements ermöglicht, eine Gewindehülse (32), die um den Hauptkörperabschnitt (22) des inneren durchbohrten Elements (12) herum befestigt ist,
einen Mandrin (14), der ein erstes Ende (36), ein zweites Ende (38) und einen länglichen Körper (40) dazwischen aufweist, wobei der längliche Körper (40) so bemessen ist, dass er durch den Innendurchgang ausreichend passt, damit das zweite Ende (38) des Mandrins (14) aus dem zweiten Ende (20) des inneren durchbohrten Elements (12) herausragt, wenn es durch die Öffnung (34) desselben eingeführt wird,
ein äußeres Hülsenelement (16), wobei das äußere Hülsenelement (16) ein erstes Ende (46), ein zweites Ende (48) und einen Hauptkörperabschnitt (50) aufweist, wobei das äußere Hülsenelement (16) ein Innengewinde aufweist, wobei das äußere Hülsenelement (16) durchbohrt ist, um einen inneren Hohlabschnitt aufzunehmen, wobei der innere Hohlabschnitt gewindemäßig mit einem Außendurchmesser des Hauptkörperabschnitts (22) des inneren durchbohrten Elements (12) in Eingriff steht, indem das Innengewinde (56) mit dem Gewinde der Gewindehülse (32) in Eingriff steht, wobei das zweite Ende (48) des äußeren Hülsenelements (16) ein Arbeitselement (58) aufweist, wobei das Arbeitselement (58) so konstruiert und angeordnet ist, dass es die Größe einer Öffnung erhöht, die durch den inneren Hohlabschnitt geschaffen wird, und eine Gewindeschneidvorrichtung mit einem spitzen Spitzenende und einem Gewindeabschnitt (70) ist, wobei die Gewindehülse (32) eine Gewindesteigung aufweist, die die gleiche wie eine Gewindesteigung des Gewindeabschnitts (70) ist, wobei das erste Ende (46) des äußeren Hülsenelements (16) einen Außenhülsengriff (57) aufweist,
wobei die Drehung des Außenhülsengriffs (57) eine lineare Bewegung des äußeren Hülsenelements (16) in Bezug auf das innere durchbohrte Element (12) bewirkt, so dass das Eindringen des äußeren Hülsenelements (16) in ein Knochenelement (84) durch Drehung des Außenhülsengriffs (57) einstellbar gesteuert wird, wobei die lineare Bewegung des äußeren Hülsenelements (16) in Bezug auf das innere durchbohrte Element (12) den gleichen Grad oder Entfernung aufweist, mit dem sich der Gewindeabschnitt (70) im Knochenelement (84) bewegt.

2. Jamshidi-Anordnung (10) nach Anspruch 1, wobei die Gewindebohrvorrichtung eine konische Spitze (68) umfasst.

3. Jamshidi-Anordnung (10) nach Anspruch 1, wobei das äußere Hülsenelement (16) linear entlang einer Längsachse (24) des inneren durchbohrten Elements (12) durch Drehen des äußeren Hülsenelements (16) gesteuert bewegt werden kann.

## Revendications

1. Ensemble jamshidi (10) comprenant :
un élément à canule interne (12) incluant une première extrémité (18), une seconde extrémité (20) et une partie de corps principale (22), une poignée d'élément à canule interne (28), adaptée pour être saisie d'une main, étant fixée sur ladite première extrémité (18) de cette dernière, ledit élément de poignée à canule interne (28) incluant une ouverture (34) positionnée pour permettre l'insertion de différents dispositifs médicaux par un passage intérieur dudit élément à canule interne, un manchon fileté (32) fixé autour de ladite partie de corps principale (22) dudit élément à canule interne (12),
un stylet (14) incluant une première extrémité (36), une seconde extrémité (38) et un corps allongé (40) entre elles, ledit corps allongé (40) étant dimensionné pour se monter à travers ledit passage intérieur suffisamment pour que ladite seconde extrémité (38) dudit stylet (14) fasse saillie de ladite seconde extrémité (20) dudit élément à canule interne (12) lorsqu'il est inséré à travers ladite ouverture (34) de ce dernier,
un élément de manchon externe (16), ledit élément de manchon externe (16) incluant une première extrémité (46), une seconde extrémité (48) et une partie de corps principale (50), ledit élément de manchon externe (16) incluant un filetage interne (56), ledit élément de manchon externe (16) présentant une canule pour comprendre une partie creuse intérieure, ladite partie creuse intérieure étant mise en prise, par filetage, sur un diamètre externe de ladite partie de corps principale (22) dudit élément à canule interne (12) en mettant en prise ledit filetage interne (56) avec le filetage dudit manchon fileté (32), ladite seconde extrémité (48) dudit élément de manchon externe (16) comprenant un élément de travail (58), dans lequel ledit élément de travail (58) est fabriqué et agencé pour augmenter la taille d'une ouverture créée par ladite partie creuse intérieure et est un dispositif de taraudage ayant une extrémité de pointe pointue et une partie filetée (70), ledit manchon fileté (32) ayant un pas de filetage qui est identique au pas de filetage de ladite partie filetée (70), ladite première extrémité (46) dudit élément de manchon externe (16) incluant une poignée de manchon externe (57),
moyennant quoi la rotation de ladite poignée de manchon externe (57) provoque le mouvement linéaire dudit élément de manchon externe (16) en référence audit élément à canule interne (12) de sorte que la pénétration dudit élément de manchon externe (16) dans un élément d'os (84) est commandée de manière ajustable par la rotation de la poignée de manchon externe (57) avec le mouvement linéaire dudit élément de manchon externe (16) en référence audit élément à canule interne (12) qui est au même taux ou la même distance au fur et à mesure que ladite partie filetée (70) se déplace à l'intérieur de l'élément d'os (84).

2. Ensemble jamshidi (10) selon la revendication 1, dans lequel ledit dispositif de taraudage comprend une pointe conique (68).

3. Ensemble jamshidi (10) selon la revendication 1, dans lequel ledit élément de manchon externe (16) peut être déplacé de manière linéaire le long d'un axe longitudinal (24) dudit élément à canule interne (12) d'une manière contrôlée en faisant tourner ledit élément de manchon externe (16).
